# EUROPEAN PATENT APPLICATION

(11) **EP 3 384 841 A1**
(43) Date of publication of application: **10.10.2018**
(21) Application number: 17164990.8
(22) Date of filing: 05.04.2017
(51) Int. Cl.: A61B 5/024, A61B 5/00

(54) **PATIENT MONITORING**

(71) Applicant: Koninklijke Philips N.V., 5656 AE Eindhoven (NL)
(72) Inventor: BOURQUIN, Yannyk Parulian Julian, 5656 AE Eindhoven (NL); KAHLERT, Joachim, 5656 AE Eindhoven (NL); CIUHU, Calina, 5656 AE Eindhoven (NL); HUIJBREGTS, Laurentia Johanna, 5656 AE Eindhoven (NL); KUENEN, Maarten Petrus Joseph, 5656 AE Eindhoven (NL)
(74) Representative: de Haan, Poul Erik

(57) **Abstract**

Presented are concepts for monitoring hemodynamics of a patient. One such concept employs a sensor unit (22) for sensing light or sound from tissue of the patient. A support unit (23) is adapted to support the sensor unit and comprises a recess (24). The support unit is adapted to be positioned against the tissue of the patient. When the support unit is positioned against the tissue of the patient, the recess is adapted to be positioned to cover a target region of the tissue of the patient and the sensor unit is adapted to detect light or sound from the target region of the tissue of the patient.

## Description

### FIELD OF THE INVENTION

This invention relates to patient monitoring, and more particular to monitoring hemodynamics of a patient.

### BACKGROUND OF THE INVENTION

Monitoring of a patient, and in particular monitoring of hemodynamics of a patient, such as cardio-respiratory function of a patient, is often relevant for an enhanced diagnostics and therapy planning for the patient. Examples of patients for which monitoring of cardio-respiratory function may be beneficial include those suffering from medical conditions such as chronic obstructive pulmonary disease (COPD), sleep-related problems (sleep disordered breathing (SDB) or insomnia, for example), respiratory disorders, asthma, and the like.

For example, the oxygenation level of blood (SO2) is an important value that may be indicative of human health status because it indicates the availability of oxygen to all tissues. Usually, SO2 refers to the oxygen saturation percentage in arterial blood (SaO2), after gas exchange in the lungs where carbon dioxide is released and oxygen is taken up.

Blood oxygenation can be measured by optical techniques, such as photoplethysmography (PPG), because oxygenated and deoxygenated hemoglobin have a different color. This can be exploited by transmitting light of two different optical wavelengths (typically at red and infrared colors) through tissue (usually the fingertip) and then measuring the light intensity with a photodiode. Such a method for measuring arterial oxygenation with PPG is called "pulse oximetry", and the arterial oxygenation measured in this way is referred to as "SpO2".

In pulse oximetry, it is assumed that attenuation in tissues, bone structures, and venous blood remains constant over time scales of a few heart beats or less, and that the observed dynamic variations in light intensity are only due to the arterial blood volume that varies with each heartbeat. These changes in light intensity (corresponding to the heart beats) give rise to an AC component of the PPG signal, while the much slower fluctuating component is a DC component of the PPG signal. To derive the SpO2 value, the relative changes in detected light (e.g. changes that are assumed to be caused by arterial blood because they pulsate due to the heart beat, as compared to the slower fluctuating component) at one wavelength (λ1) are compared to those at another wavelength (λ2), with other absorption characteristics for oxygenated and deoxygenated hemoglobin, resulting in the following ratio of ratios (RR): RR = (AC/DC)_{λ1} / (AC/DC)_{λ2}.

This ratio is subsequently used in a formula to derive the arterial oxygenation. Often, a linear relationship is assumed: SpO2 = a·RR+b, where a and b are calibration constants.

The oxygenation level in the venous system (SvO2) is often overlooked. However, whereas SaO2 (i.e. oxygenation level in arterial blood) describes the availability of oxygen to tissues, SvO2 (i.e. the oxygenation level in venous blood) allows the derivation of how much oxygen is actually taken up in the organs and tissues. SvO2 is, therefore, a clinically relevant parameter in several settings (e.g. in patient monitoring during surgery procedures). Together, SaO2 and SvO2 provide highly relevant clinical information in a wide range of settings.

Similarly to SaO2, SvO2 can be measured by inducing a venous blood volume change and subsequently measuring the oxygen saturation from optical signals at two different wavelengths. In comparison to SaO2, however, the measurement of SvO2 is more complicated, mainly for the following two reasons: (i) SaO2 is much more easily defined than SvO2, since all blood in the arteries of the systemic circulation essentially have the same oxygenation level, as the blood is distributed through all systemic arteries before any gas exchange takes place. On the contrary, venous blood is first collected from the microcirculation and eventually pooled together in the right atrium. This severely constrains the choice of measurement sites for obtaining the central venous oxygenation, and has resulted in SvO2 measurement concepts that are intrusive in nature; and (ii) There is no natural mechanism (or 'venous' equivalent of the beating heart) showing up as clearly in the signal as the pulse does for arterial blood to distinguish venous blood from arterial blood, bones, and tissues. This makes it difficult to infer SvO2 based on measured light absorption spectra.

Thus, there exists a need for non-invasive or unobtrusive monitoring concepts for monitoring cardio-respiratory function of a patient. Further, it would be particularly advantageous for concepts to be suitable for obtaining venous information (such as venous oxygen saturation, SvO2). Such non-invasive and/or unobtrusive monitoring concepts may be beneficial for enhanced diagnostics and therapy planning in relation to many medical conditions.

### SUMMARY OF THE INVENTION

The invention aims to at least partly fulfil one of the aforementioned needs. To this end, the invention provides devices, methods, computer program products and systems as defined in the independent claims. The dependent claims provide advantageous embodiments.

Thus, the invention provides an apparatus and a corresponding method for monitoring hemodynamics of a patient. Embodiments of the apparatus may comprise: a sensor unit adapted to detect light or sound from tissue of the patient and to generate sensor output signals based on the detected light or sound; and a support unit adapted to support the sensor unit and comprising a recess, wherein the support unit is adapted to be positioned against the tissue of the patient, and wherein, when the support unit is positioned against the tissue of the patient, the recess is adapted to be positioned to cover a target region of the tissue of the patient and the sensor unit is adapted to detect light or sound from the target region of the tissue of the patient.

Proposed embodiments are based on arranging a sensor unit in a support structure that may be positioned against tissue of a patient so that the sensor unit may be optimally situated for detecting light and/or sound from the patient's tissue. The support structure may be adapted so that, when it is placed against tissue of a patient, a target region of tissue is not excessively squeezed or compressed. Also the support structure may be arranged to support the sensor unit so that it may detect light or sound from the target region of the tissue, thereby enabling accurate measurements to be obtained.

For example, by providing a recess in the support unit that is adapted, in or during use, to be positioned so as to cover a target region of a patient's tissue (such as a region of tissue containing a target vein), the support unit may be prevented from pressing down on the target region of the patient's tissue (which may otherwise cause veins in the tissue to collapse for example). Also, the sensor unit may be provided in the recess and, in this way, the sensor unit may be offset (e.g. 'set back from') from the locations at which the support structure contacts the patient's tissue, thereby preventing the sensor from pressing down on the patient's tissue (which may otherwise cause veins in the tissue to collapse for example). For instance, the support unit may be adapted such that when it is positioned against the tissue of the patient: the support unit contacts the tissue at first and second contact regions situated either side of the target region of the tissue of the patient; and the sensor unit is offset from a plane passing through the first and second contact regions.

Put another way, a recess, trench, ditch or channel may be provided in a structure that is designed to be positioned on the patient's tissue. By positioning the sensor unit in the recessed portion (e.g. recess, trench, ditch or channel) of the support structure, the sensor unit may be arranged not to press down excessively on a target region of the tissue (when the recessed portion is positioned so as to cover the target region of the tissue). The recessed portion may, instead, provide a space for the target region of the tissue to enter when the structure is placed on the tissue, thereby preventing the target region of the tissue from being compressed (which may cause an underlying vein to collapse for example).

For example, when the support unit is positioned against the tissue of the patient, the recess and the target region of the tissue of the patient may combine to form a chamber. Such a chamber may provide room for a vein underlying the tissue, thereby preventing the vein from being collapsed (which would otherwise block blood flow). Also, the sensor unit may be positioned so that it faces the target region of the tissue of the patient when the chamber is formed. For example, the support unit may support the sensor unit such that, when the support unit is positioned against the tissue of the patient, the sensor unit and the target region of the tissue are positioned at opposite side of the chamber.

Further, in some embodiments, a vacuum arrangement may be employed which is adapted to create a negative pressure in the chamber when the support unit is positioned against the tissue of the patient. A negative pressure or vacuum in the chamber may cause the surface of the tissue to be brought into contact with (or towards) the sensor unit, thereby minimizing or removing any air gap between the tissue and the sensor unit for improved sensing accuracy.

By way of example, the support unit may comprise a recess that is arranged such that, when the support unit is positioned against the tissue of the patient, the recess may be positioned to cover the target region of the tissue of the patient. The recess may therefore provide a volume for the target region of the tissue to enter so it is not unduly compressed when the support unit is positioned against the tissue of the patient. For instance, the target region of the tissue may comprise a vein and so the recess may be adapted to have a depth that is substantially equal to the diameter of the vein. Such an arrangement may prevent the vein from being collapsed whilst still enabling the surface of the tissue to contact the sensor unit in the chamber for example. For example, for sublingual veins, a typical diameter of a vein is about 2.7 mm. The recess may therefore provide with a width of 2 to 5 mm (and preferably about 3 mm) and a height/depth of 1 to 4 mm (and preferably about 2 mm). For instance, if the recess is provided in the form of a gutter having a cross-sectional shape of a semi-circle (e.g. a gutter having a shape of a half-cylinder), the radius of the cross-sectional shape of the gutter may be in range of 1 to 2.5 mm (and preferably about 1.5 mm).

A signal from the sensor unit that is representative of detected light or sound from the sublingual vein may then be used to provide information on cardio-respiratory parameters like venous pulsation, venous blood accumulation, respiration rate (RR), respiration rate variability (RRV), onset of inspiration, onset of expiration, duty cycle of respiration, Cheyne-Stokes respiration, obstructive sleep apnea (OSA), central sleep apnea (CSA), distinction between OSA and CSA, obstructive flow limitation (Hypopnea), mean transmural pressure, mean blood accumulation, the presence of edema, sighing, yawning, coughing, paced breathing, and pursed breathing.

Accordingly, there may be provided a tool for determining and/or monitoring a cardio-respiratory information that can be used by a medical professional, a general practitioner without the support of a trained cardiologist, for example. It may assist in the diagnosis of dynamic changes of the preload and blood accumulation in heart failure patients. Similarly, it may help to diagnose and stratify persons who are at the onset of developing heart failure and pulmonary edema. Embodiments may also be used to verify the success of a cardiac therapy and/or to monitor disease progression or exacerbation.

Also, embodiments may be used for detecting or monitoring sleep apnea in a sleep study. For example, proposed embodiments may be employed to detect obstructive and central apnea and may distinguish between these. Further, an embodiment may be used to monitor the cardio-respiratory response of a ventilation therapy and to better control the device settings of a pressure support therapy in COPD and OSA patients.

By way of example, embodiments may be used by a general physician or (medically) un-trained person without the support of a trained cardiologist. This may alleviate a need for close monitoring by medical professionals. It may also reduce a need for medical intervention or treatment. Embodiments may therefore relieve healthcare requirements/resources.

The support unit may be adapted so as to enable the sensor unit to be positioned at vasculature draining into the internal jugular vein of the patient. For example, the support unit and sensor unit may form or provide a sublingual optical sensor unit adapted to be positioned at the sublingual vasculature of the patient's tongue, to detect light from the sublingual vasculature and to generate the sensor output signals based on the detected light. For instance, the support unit may be adapted to be positioned at the sublingual vasculature of the patient's tongue, and the target region of the tissue of the patient may comprise the sublingual vasculature. Thus, some embodiments may be based on using the sublingual vein and/or sublingual vein proximity vasculature of a patient's tongue to look at the venous blood volume/flow and that the variations in blood volume/flow in the sublingual vein or vasculature will give information on cardio-respiratory interactions. In this regard, it should be noted that probing only the sublingual vein might not be possible (because the positioning comes very critical) and it might not be desirable either. When an area is probed that is only a very short distance from the sublingual vein, the venules still give the desired signal representative for the respiratory- and blood accumulation-related information and next to that, information is obtained from the arterioles in that area, from which the heart rate, heart rate variability and arterial blood oxygenation can be obtained. For those reasons, when herein reference is made to 'positioned at a sublingual vein', 'aimed at the sublingual vein', or 'sublingual vasculature', it should be understood to include being at an area which is a close distance from (i.e. proximate to) the sublingual vein wherein information from the venules that drain in the sublingual vein can be derived from the signal. This is preferably at a distance less than 1 cm from the sublingual vein, and more preferably at a distance less than 5 mm from the sublingual vein. In this way, it will be understood that, in proposed embodiments, a sublingual sensor unit may be adapted to be positioned at, aimed at, or proximate the sublingual vein or sublingual venous vasculature of the patient's tongue.

The advantage of the sublingual vein is that it lies close to the surface and that the sublingual skin is very thin and optically transparent. Also, the bottom side of the tongue is a highly perfused area. Those properties make the bottom side of the tongue well-suited for optical monitoring for example. Further, compared to many other veins or vasculature in other places in the body, the sublingual vein or vasculature is of special interest because it drains into the internal jugular vein, which then drains into the vena cava; there is therefore a close connection to the central veins and the right side of the heart. Especially in lying position information for the right side of the heart and the central veins can be seen derived from the sublingual vein.

It may therefore be proposed to arrange or adapt a sensor unit to be aimed at the sublingual vasculature/area on the bottom side of a patient's tongue. This may, for example, enable more of the venous component to be seen in the sensor signals. Proposed embodiments may therefore leverage the raw signal of the sensor.

In other embodiments, other sites with veins draining into the internal jugular vein are probed. Examples are the nose, the eye, the inner ear and the forehead.

Signals from the optical sensor unit that are representative of detected light from the tissue may be used to provide information on cardio-respiratory parameters like respiration rate (RR), respiration rate variability (RRV), onset of inspiration, onset of expiration, duty cycle of respiration, Cheyne-Stokes respiration, obstructive sleep apnea (OSA), central sleep apnea (CSA), distinction between OSA and CSA, obstructive flow limitation (Hypopnea), mean transmural pressure, mean blood accumulation, the presence of edema, sighing, yawning, coughing, paced breathing, and pursed breathing.

The sensor unit may be used either in reflective or transmissive mode.

In an embodiment, the sensor unit may comprise at least one of: a PPG sensor; a laser speckle sensor; a laser Doppler sensor; an ultrasound sensor; and a camera. It is noted that that PPG sensors exist that, when placed on the finger or ear lobe, may enable the derivation of RR from the sensor signal. However, for sensors placed at these locations, it is practically impossible to see the RR by eye from the raw PPG signal. Therefore, RR can only be derived with relatively complex algorithms, which generally take into account modulations in frequency, amplitude and DC-level. Even then, the derived RR is not always correct. In contrast, the raw PPG signal of a PPG sensor aimed at the sublingual vein in accordance with proposed embodiment clearly shows RR, onset of inspiration, onset of expiration and depth of inspiration thereby potentially avoiding the need for complex algorithms and/or extensive processing resources.

Embodiments may further comprise a light source adapted to illuminate the body tissue of the patient. The light source may be adapted to emit first light having a wavelength within a first range of wavelengths and to emit second light having a wavelength within a second, different range of wavelengths, or more than one light sources might be used, which each have their own specific wavelength band. By way of example, the first range of wavelength may comprise visible light and the second range of wavelengths may comprise infra-red light. In order to derive SO2, red and infra-red light are commonly used. Since blood in the veins and venules contains more deoxygenated blood than blood in the arteries and arterioles and since red light is absorbed substantially more by deoxygenated blood than by oxygenated blood (while the absorption in infra-red is similar), red light is especially suitable to derive venous information. In order to have a further distinction between venous and arterial information, the red signal and the infra-red signal could be compared, e.g. the infra-red signal might (preferably after weighing) be subtracted from the red signal.

In some embodiments, an ultrasound transducer or ultrasound transceiver may be employed to transmit and sense ultrasound signals to/from the patient's tongue.

Embodiments may further comprise an output interface adapted to generate an output signal representative of determined or calculated venous information. For example, a user may be advised of a cardio-respiratory value exceeding a predetermined acceptable threshold.

Embodiments may further comprise a user input interface adapted to receive a user input signal representative of at least one of: environmental information; patient information; and a limit value representative of an acceptable upper limit of a cardio-respiratory value. Embodiments may therefore be thought of as providing an interface which enables a user to further specify information or data that may be relevant for the purpose of determining or monitoring a cardio-respiratory value. Such user-specified information may enable unique traits, circumstances and/or conditions specific to the user or the environment to be accounted for when determining or monitoring a cardio-respiratory value.

Thus, there may be provided a tool which enables a user to further specify factors to be included in the determination or monitoring of cardio-respiratory function., e.g. by specifying a value or value range for a user attribute or activity. Embodiments may therefore provide input options, increasing the flexibility and power of risk of cardio-respiratory monitoring.

In some embodiments, the apparatus may further comprise a communication interface adapted to communicate with one or more databases so as to obtain at least one of the information that may be used in determining or monitoring a cardio-respiratory value.

There may be provided a portable computing device comprising apparatus for monitoring a cardio-respiratory function of a patient according to a proposed embodiment.

The system may further comprise a display device for displaying a graphical or non-graphical (e.g. auditory) user interface, wherein the graphical user interface is adapted to communicate information about detected or monitored cardio-respiratory function of a patient to a user.

Embodiments may comprise a client device comprising a data processor device. This may be a standalone device adapted to receive information from one or more remotely positioned information sources (via a communication link for example) and/or even adapted to access information stored in a database for example. In other words, a user (such as a medical professional, technician, researcher, patient etc.) may have an appropriately arranged client device (such as a laptop, tablet computer, mobile phone, PDA, etc.) which provides a system according to an embodiment and thus enables the user to provide data or information for the purpose of monitoring cardio-respiratory function of a patient.

The system may comprise: a server device comprising the at least one processor, where the server device may be configured to transmit generated instructions for determining and/or displaying a cardio-respiratory function of a patient to a client device or communication network. In such a configuration, display instructions are made available by a server. A user may therefore link with the server to work with the system.

The processor may be remotely located from the display device, and a control signal may thus be communicated to the display device via a communication link. Such a communication link can be e.g. the internet and/or a wireless communication link. Other suitable short-range or long-range communication links and/protocols may be employed. In this way, a user (such as a medical researcher, general practitioner, data analyst, engineer, patient etc.) may have an appropriately arranged device that can receive and process information according to an embodiment for monitoring a cardio-respiratory function of a patient. Embodiments may therefore enable a user to remotely monitor cardio-respiratory function of a patient using a portable computing device, such as a laptop, tablet computer, mobile phone, PDA, etc. Embodiments may also enable data retrieval after a monitored time period.

The system may further comprise: a server device comprising the at least one processor; and a client device comprising a display device. Dedicated data processing means may therefore be employed for the purpose of monitoring a cardio-respiratory function or value of a patient, thus reducing processing requirements or capabilities of other components or devices of the system.

Thus, it will be understood that processing capabilities may therefore be distributed throughout the system in different ways according to predetermined constraints and/or availability of processing resources.

Embodiments may further comprise a supplementary sensor module (e.g. a supplementary sensor adapted to be positioned in the patient's mouth) comprising a sensor arrangement adapted to sense a value of at least one of: movement; pressure; temperature; and sound and to generate a supplementary sensor output signal based on the sensed value(s). For example, a supplementary PPG sensor may be provided and adapted for use at a different location of the patient (e.g. adapted to probe arterial blood). In such embodiments, the supplementary sensor may provide additional information about the patient which may, for example, be useful for improving accuracy of determinations or refining previously obtained values.

In an embodiment, the apparatus may further comprise a processing unit adapted to receive at least one of the sensor output signal and the supplementary sensor output signal and to process at least one of the received signals in accordance with one or more data processing algorithms to determine a cardio-respiratory value of the patient.

By way of example, the cardio-respiratory value may comprise a value of at least one of: respiration rate; respiration rate variability; onset of inspiration; onset of expiration; duty cycle of respiration; Cheyne-Stokes respiration; obstructive sleep apnea; central sleep apnea; obstructive flow limitation; transmural pressure; blood accumulation; coughing; paced breathing; and pursed breathing.

Typical sampling frequencies of the sensor signal may be 32, 128 or 256 Hz, but the sampling frequency might also have a different value. The data-processing algorithm may be adapted to identify low-frequency variations in the sensor output signal, to get information indicative for the respiration. For example, a RR may be observed as the dominant frequency in the range between. 0.08 Hz and 0.5 Hz. In PPG, this frequency band and lower frequencies are referred to as 'DC', as they are lower than the changes caused by heart rate. Changes in the depth of respiration can, for example, be interpreted from amplitude variations of the PPG signal at the respiration frequency. Embodiments may monitor modulation in a low frequency sensor signal component which indicate the signal response to the deviations in respiration (caused by additional stress in the heart for example). Nevertheless, higher frequencies variations in the sensor output signal (for example in the range between 0.6 Hz and 4 Hz) may also be used to provide information on average heart rate. In PPG, fluctuation in the signal caused by the pulsations of the heart are called 'AC' fluctuations. Heart rate variability and arrhythmias may be derived from a highpass-filtered signal with a threshold frequency of e.g. 0.5 or 0.6 Hz. SpO2 may be derived from both AC and DC components, while RRV may require determination of the duration of each breath.

The processing unit may be adapted to receive at least one of the sensor output signal and the supplementary sensor output signal, to process the received sensor output signal in accordance with a data processing algorithm to determine a cardio-respiratory value of the patient, and to analyze the determined cardio-respiratory value in combination with the received supplementary sensor output signal to determine at least one of: a refined cardio-respiratory value; an indication of accuracy or reliability; a sleep state of the patient; an activity of the patient; and an indication of event occurrence. Embodiments may therefore take account of a context of the patient, such as their current activity or physical attributes for example.

According to another aspect of the invention, there may be provided a mouthpiece (or oral appliance) comprising apparatus for monitoring cardio-respiratory function of a patient according to a proposed embodiment. For example, embodiments may propose the use of a sensor supported in an oral appliance, wherein the oral appliance on the lower teeth houses or supports the sensor. In this way, the sensor may be stably positioned in the mouth, thereby reducing motion artefact. In some embodiments, the sensor may be adapted to be movable with respect to the oral appliance (e.g. under the control of a mechanical or electro-mechanical arrangement), thereby enabling the positioning of the sensor unit to be optimized or personalized to a specific-patient for example.

According to yet another aspect of the invention, there may be provided a method for monitoring hemodynamics of a patient, the method comprising: positioning a support unit against the tissue of the patient, wherein the support unit comprises a recess and supports a sensor unit, the sensor unit being adapted to detect light or sound from tissue of the patient and to generate sensor output signals based on the detected light or sound, and wherein when the support unit is positioned against the tissue of the patient, the recess is adapted to be positioned to cover a target region of the tissue of the patient; detecting light or sound from the target region of the tissue of the patient; and generating a sensor output signal based on the detected light or sound.

In some embodiments, when the support unit is positioned against the tissue of the patient, and the recess and the target region of the tissue of the patient may combine to form a chamber with the sensor unit being positioned to face the target region of the tissue of the patient. Also, in an embodiment, the method may further comprise creating a negative pressure in the chamber.

Embodiments may further comprise the step of illuminating the patient's tongue with light from a light source. This, may for example comprise: controlling the light source to emit light having a wavelength within a first range of wavelengths and to emit light having a wavelength within a second, different range of wavelengths. Preferably, the first range of wavelength may comprise visible light and the second range of wavelengths may comprise infra-red light. The light source may thus comprise one or more light emitting devices.

Embodiments may provide concepts for monitoring one or more cardio-respiratory functions of a patient. The proposed concepts may comprise positioning a support unit (supporting a sensor) at (e.g. adjacent, proximate, next to, neighboring, etc.) a sublingual vein of the patient's tongue. Light or sound transmitted through the sublingual vein may be detected by the supported sensor and the detected light or sound may be used (e.g. processed according to one or more algorithms) to determine a value of a cardio-respiratory functions of the patient. Determining the value of a cardio-respiratory function may comprise taking account of historical information relating to previously determined values of the cardio-respiratory function of the patient. Further, additional sensors may be employed to detect one or more supplementary values of other physical attributes or parameters of the patient. Such supplementary values may be used in combination with the detected light and/or determined value of the cardio-respiratory function to infer or determine other information (such as an indication of accuracy or reliability, a sleep state of the patient, an activity of the patient, or an indication of event occurrence for example) and/or confirm/validate the determined value of the cardio-respiratory function. For this purpose, proposed concepts may employ (or be employed on) at least one processor.

Proposed embodiments may further comprise generating instructions for displaying a GUI on a display device using a processor device, wherein the graphical user interface is adapted to communicate information about detected light or sound from the sublingual vein and/or a determined cardio-respiratory value of the patient to a user. Generating instructions for display of a GUI can mean generating a control signal for use by a display device. Such instructions can be in the form of simple images such as bitmap JPEG or other format. However, such instructions can also be more complex allowing real time build-up of the GUI or parts of the GUI on a regular display device such as for example CRT, LCD, OLED, E-ink or the like.

According to another aspect, there may be provided a computer program product downloadable from a communications network and/or stored on a computer readable medium and/or microprocessor-executable medium wherein the computer program product comprises computer program code instructions, which when executed by at least one processor, implement a method according to a proposed embodiment.

The independent claims define analogous advantages features for method and system claims. The advantages explained for the method herein above and herein below may therefore also apply to the corresponding systems.

### BRIEF DESCRIPTION OF THE DRAWINGS

The invention will now be described in detail with reference to the following schematic drawings:
Fig. 1A illustrates an apparatus for monitoring cardio-respiratory function of a patient according to an embodiment;
Fig. 1B depicts the sublingual apparatus of Fig. 1A wherein a vein is positioned in the recess so as to illustrate how the recess provides a space for receiving a receiving a vein when the apparatus is positioned against tissue of a patient;
Fig. 1C depicts a cross-sectional view of the apparatus of Fig. 1B taken along the line X-X in Figure 1B;
Fig. 1D depicts a modification to the embodiment illustrated in Fig. 1C;
Fig. 2 illustrates an apparatus for monitoring hemodynamics of a patient according to an embodiment;
Fig. 3 is a flow diagram of a method for monitoring hemodynamics of a patient according to an embodiment; and
Fig. 4 illustrates an example of a computer system within which one or more parts of an embodiment may be employed.

### DETAILED DESCRIPTION OF THE EMBODIMENTS

Proposed embodiments relate to approaches and tools for monitoring hemodynamics or cardio-respiratory function of a patient. A support unit may be employed to support a sensor unit, and the support unit may be employed (e.g. positioned) at the patient's body tissue so as to detect light or sound from a target region of the tissue. The support unit may be adapted so that it does not excessively compress, squash, squeeze or collapse the target region of the tissue while the sensor unit is positioned close to, or in contact with, the target region so that it can detected light or sound from the target region of the tissue. Sensor output signals may then be generated based on the detected light or sound. The signals may be used (e.g. processed) to determine a value of cardio-respiratory parameter of the patient.

Embodiments are therefore based on using a device which combines a housing or support structure with a sensor unit in order to enable the sensor unit to be positioned at, against or on vasculature of a patient to detect light or sound from the vasculature while preventing the vasculature (e.g. a vein) from being compressed or collapsed. The housing or support structure may provide a chamber, recess or gutter which is adapted to be positioned against a patient's tissue and cover a target region of the tissue (e.g. a region of tissue comprising a target vein) so that the chamber, recess or gutter provides a space above the target region so that the target region of the tissue is not pressed-down upon and compressed/collapsed in use. The sensor unit can be provided in the recess so that, in use, it is positioned close to or touches the tissue without collapsing blood flow in the target region of the tissue.

By way of example, embodiments may be adapted to use the sublingual vein or vasculature of a patient's tongue to detect variations in blood volume/flow in the sublingual veins/vasculature. Such an approach leverages the fact that the sublingual vein lies close to the sublingual (i.e. bottom or underside) surface of the tongue and that the sublingual skin is thin and optically transparent. Such properties make the sublingual side of the tongue highly-suited for detecting and monitoring blood flow or volume variations.

By way of example, a signal from the sensor unit may be used to provide information on cardio-respiratory parameters including: RR, RRV, onset of inspiration, onset of expiration, duty cycle of respiration, Cheyne-Stokes respiration, OSA, CSA, distinction between OSA and CSA, Hypopnea, mean transmural pressure, mean blood accumulation, the presence of edema, sighing, yawning, coughing, paced breathing, and pursed breathing, for example.

Embodiments may therefore be utilized to monitor the cardio-respiratory response of a ventilation therapy and to better control device settings of a pressure support therapy in COPD and OSA patients.

Also, the proposed invention may provide concepts for monitoring one or more cardio-respiratory parameters that can be employed by a general physician or (medically) un-trained person without the support of a trained cardiologist. This may alleviate a need for medical professionals and/or medical intervention, thus potentially relieving healthcare requirements/resources.

When the support unit is positioned against the tissue of the patient, the recess and the target region of the tissue of the patient may combine to form a chamber. Such a chamber may provide room for a vein underlying the tissue, thereby preventing the vein from being collapsed (which would otherwise block blood flow).

Further, in some embodiments, a vacuum or negative pressure may be created in the chamber when the support unit is positioned against the tissue of the patient. The negative pressure or vacuum in the chamber may bring the patient's tissue into contact with (or towards) the sensor unit, thereby minimizing or removing any air gap between the target region of the tissue and the sensor unit for improved sensing accuracy.

Some embodiments may employ a supplementary sensor module. For example, the supplementary sensor module may comprise a sensor arrangement adapted to sense: movement; pressure; temperature; and/or sound and to generate a supplementary sensor output signal based on the sensed value(s). For example, information regarding sensed motion may be useful for indicating signal quality and/or reliability. Embodiments of the invention may therefore be utilized in conjunction with many different types of additional sensors and/or information databases that may provide contextual information useful for determining a patient's cardio-respiratory function and which more accurately accounts for the specific attributes of the patient, activity of the patient, and/or surrounding environment. A database may comprise, for instance, data relating to the individual's medical history or data relating to cardio-respiratory parameter values in different environmental conditions. For example, information or data employed by embodiments may comprise patient activity, vital signs, temperature, etc.

Embodiments may therefore provide a method, device and/or system that provides for user-specific assessment and monitoring of cardio-respiratory function(s) which takes account of contextual factors (including a physical attributes and activity of a patient, for example) in order to provide more accurate assessment and tracking of cardio-respiratory function or parameter. This may enable measurement and tracking of cardio-respiratory for a specific user, whilst enabling the user to partake in desired activities of daily life.

Illustrative embodiments may therefore provide concepts which take account of rules and/or relationships relating to activity and physical attributes of the patient. Dynamic context-based venous information detection and monitoring may therefore be provided by proposed embodiments. In particular, embodiments may communicate information about the venous information in a simple manner (e.g. by visual and/or audible alert) so that a user can readily and easily understand their personal cardio-respiratory function.

As a result, proposed embodiments may be of benefit in any cardio-respiratory function assessment or monitoring applications, especially where users require tailored and/or accurate determination of venous oxygen saturation. One such example may enable patients that are highly susceptible to cardio-respiratory problems to gain a level of independence whilst still managing their potential exposure to cardio-respiratory issues. This may, in turn, improve patient health, hospital efficiency, and available healthcare resources. Embodiments may therefore be of particular benefit for medical applications.

The following description provides a context for the description of elements and functionality of the invention and of how elements of the invention can be implemented.

In the description, the following terms and definitions are used.

A graphical user interface (GUI) is a type of interface that allows users to interact with electronic devices through graphical icons and visual indicators such as secondary notation.

A display device is an electronic display device that can be controlled by a display control device. The display control device can be part of, or operate together with, a processor device.

Generating instructions for displaying a GUI can comprise (or be as simple as) constructing images (Bitmap, JPEG, Tiff or the like) of GUI views to be displayed on a display device using regular methods known in the art. Alternatively, such generation of instructions can comprise more dedicated instructions for real time build-up of a GUI view. The instructions can be in the form of a display control signal.

The invention is at least partly based on the insight that it is advantageous to provide a structure that is adapted to provide a space (e.g. a recess or channel) above a target region of tissue when the structure is placed onto the tissue of a patient. This space may prevent the target region of tissue from being squashed, compressed or squeezed. Also, by supporting a sensor unit on the structure such that the sensor unit is adjacent (i.e. next to, on or at) the target region and offset from a plane passing through points of tissue contact on opposite sides of the target region, the sensor may be positioned so that it does not compress or press down on the target region of tissue.

In other words, use of a support device/structure to support a sensor unit and be placed at or against a vasculature may enable the sensor unit to be positioned to detect light or sound from a target region of vasculature without the vasculature being compressed to an extent that prevents or alters blood flow in the target region.

This may be particular advantageous for use at the sublingual vein of a patient's tongue so as to determine a value of cardio-respiratory parameter of the patient. In particular, detecting light from the sublingual vein in response to the tongue being illuminated by a light source can be used to accurately determine a cardio-respiratory parameter of the patient. In other words, use of a sublingual optical sensor unit placed at or against a sublingual vein of the patient's tongue so as to detect light from the sublingual vein may be used (e.g. processed) to determine a value of cardio-respiratory parameter of the patient. Also, detecting sound from the sublingual vein may be used to accurately determine a cardio-respiratory parameter of the patient. For instance, an ultrasound transducer may be employed for converting electrical signals into high-frequency sound waves and vice-versa. In other words, use of a sublingual ultrasound transceiver placed at or against a sublingual vein of the patient's tongue so as to detect reflected sound waves from the sublingual vein may be used (e.g. processed) to determine a value of cardio-respiratory parameter of the patient.

It will be appreciated that the accuracy of cardio-respiratory parameter determination will depend on the amount and quality of data used. Proposed embodiments may provide data of improved quality by enabling a sensor unit to be positioned at vasculature using a support structure that prevents a target region of the vasculature from being compressed by the sensor unit.

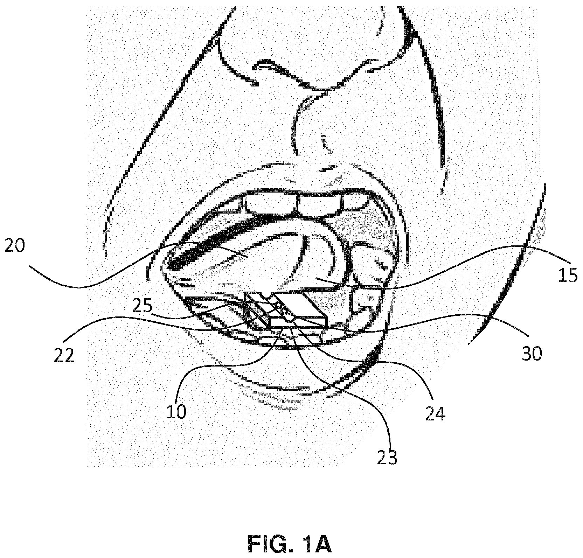

According to various embodiments, there are proposed several approaches to monitoring cardio-respiratory function of a patient. Turning firstly to Figure 1A, there is depicted an embodiment wherein sublingual apparatus 10 is positioned at a sublingual vein 15 of the patient's tongue 20.

The sublingual apparatus 10 comprises an optical sensor unit 22 and a support unit 23.

The support unit 23 is adapted to support the optical sensor unit 22 and to be positioned against the underside (e.g. the sublingual vasculature 15) of patient's tongue 20 so as to contact the tissue at first and second contact regions situated either side of a target region of the tissue of the patient.

Here, the support unit 23 comprises a recess 24 in the form of a gutter or elongate trench. When the support unit 23 is positioned against the underside (e.g. the sublingual vasculature 15) of patient's tongue 20, the recess 24 is adapted to be positioned to cover the target region of the tissue underside (e.g. the sublingual vasculature 15) of the patient. Here, because there are two main veins under the tongue 20 (e.g. a main vein on either side), the recess is not positioned along a central axis of the support unit 23, but is instead positioned to one side of the central axis. In other embodiments, however, two recesses may be provided, one for each main vein).

Also, the optical sensor unit 22 is provided in the recess 24 so that, when the support unit 23 is positioned against the underside (e.g. the sublingual vasculature 15) of patient's tongue 20, the optical sensor unit 22 is offset from a plane passing through the first and second contact regions. In this way, the optical sensor unit 22 is arranged to detect light from the target region of the tissue (e.g. the sublingual vasculature 15) of the patient.

In particular, when the support unit 23 is positioned against the underside (e.g. the sublingual vasculature 15) of patient's tongue 20, the recess 24 and the underside of patient's tongue 20 combine to form a chamber with the optical sensor unit 22 and the target region of the tissue (e.g. the sublingual vasculature 15) of the patient being positioned at opposite sides of the chamber. Put another way, when the support unit 23 is positioned against the underside (e.g. the sublingual vasculature 15) of patient's tongue 20, the support unit 23 may position the optical sensor 22 so that it is slightly spaced apart from and facing the target region comprising sublingual vasculature 15 of the patient. Preferably, the spacing between the optical sensor 22 and the sublingual vasculature 15 may be as small a practically possible and more preferably zero (so that optical sensor 22 is just touching the sublingual vasculature 15 and exerting a minimal or near-zero compressive force on the sublingual vasculature for example).

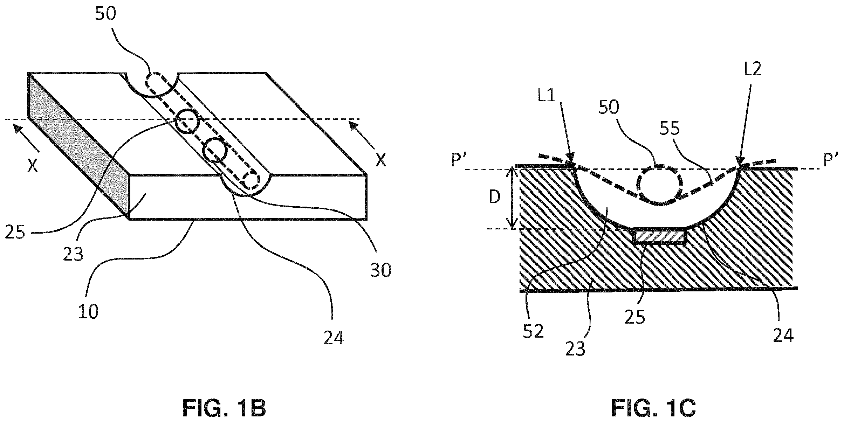

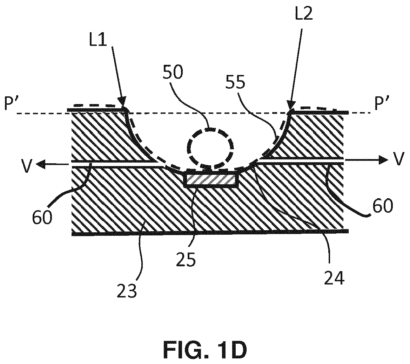

In this embodiment, the optical sensor unit 22 comprises an optical sensor 25 and a light source 30. The light source 30 is adapted to illuminate the underside (e.g. the sublingual vasculature 15) of patient's tongue 20. More specifically, the light source 30 of this embodiment is adapted to emit light of two differing wavelength ranges, namely a first light having a wavelength within a first range of wavelengths and a second light having a wavelength within a second, different range of wavelengths. Here, the first range of wavelength comprises red light (or light having a wavelength towards the red end of the visible light spectrum) and the second range of wavelengths comprises infra-red light. Of course, in other embodiments, the light source may only emit one type (e.g. wavelength range) of light and/or may emit light of wavelengths different to this example of Figure 1. As another alternative, near-infrared spectroscopy (NIRS) may be used instead of multi-wavelength PPG.

The optical sensor 25 is a photoplethysmography (PPG) sensor and is adapted to detect light from the sublingual vein in response to the illuminating the patient's tongue with light from the light source 30. Thus, in this example the optical sensor unit 22 may be said to operate in a "reflective mode", because the optical sensor 25 and the light source 30 are both situated under the tongue at the sublingual vein 15. Light from the light source 30 therefore illuminates the sublingual vein 15 of the patient's tongue 20 from underneath the tongue 20 and the light reflected by the sublingual vein 15 is then detected by the optical sensor 25. Other examples may, however, employ an optical sensor unit 22 that is said to operate in a "transmissive mode", wherein the optical sensor 25 is situated under the tongue at the sublingual vein 15 and the light source 30 is situated above (e.g. at the upper surface) of the tongue so as to illuminate the tongue from above, or, the opposite situation, wherein the optical sensor 25 is situated on top of the tongue and the light source 30 is situated below the tongue. In such a transmissive mode, the light source 30 illuminates the patient's tongue 20 and the light transmitted through the patient's tongue 20 is then detected by the optical sensor 25.

Based on the detected light, the optical sensor 25 generates a sensor output signal for outputting to a signal processing unit. In this embodiment, the signal processing unit is not integrated into the sublingual apparatus 10, but is instead provided as part of a portable computing device (e.g. a smartphone) 100. Of course, in other embodiments, the signal processing unit may be integrated in the sublingual apparatus 10.

By way of example only, the signal processing unit of this embodiment is provided as a wearable or clip-on computing device that is worn or carried by the patient. Thus, for communicating the sensor output signal to the signal processing unit, the sublingual apparatus 10 comprises a communication interface (not shown) which is adapted to establish a wireless communication link with the signal processing unit. Any suitable short-range or long-range communication links and/protocols may be employed.

The signal processing unit receives the sensor output signal processes the received signal in accordance with a signal processing algorithms to determine a cardio-respiratory value of the patient. More specifically, the sensor output signal in this embodiment is a raw PPG signal of the PPG sensor 25 and clearly shows RR, thereby potentially avoiding the need for complex algorithms and/or extensive signal processing resources.

The determined cardio-respiratory value of the patient can be communicated to the patient in many different. For example, the patient can be warned if a determined cardio-respiratory value is indicative of a problem or issue (e.g. a cardio-respiratory value is increasing rapidly), and/or determined values for the cardio-respiratory parameter for a predetermined time period (e.g. current day, the past 24 hrs., or a longer period) can be shown to the user, and a warning can be provided if a threshold is reached.

Data obtained for patient can also be shared with other users as a form of community data. However, it is noted that sharing data on identifiable persons as potential infection sources may give rise to privacy issues.

Referring now to Figure 1B, the sublingual apparatus 10 of Figure 1A is depicted wherein a vein 50 is positioned in the recess 24 so as to illustrate how the recess 24 provides a space for receiving a receiving a vein when the apparatus 10 is positioned against tissue of a patient. Here, the vein 50 is depicted using dashed lines and the concept of arranging the cross-sectional area of the recess 24 to be larger than the cross-sectional area of the vein 50 is shown by the relative sizing of the recess 24 and the vein 50. Of course, it will be understood, however, that the relative sizing of the recess 24 and the vein 50 may not necessarily be accurately depicted in Figure 1B.

From Figure 1B it will be appreciated that, when the support structure 23 is positioned against tissue of a patient, a vein 50 in a target region of tissue is not excessively squeezed or compressed (e.g. because the vein 50 is positioned in the space provided by the recess 24). Also, the support structure 23 supports the optical sensor 25 and the light source 30 so that they are positioned close to (or even touching) the vein so that the optical sensor 25 may detect light from vein 50, thereby enabling accurate measurements to be obtained.

Referring to Figure 1C (which depicts a cross-sectional view of the apparatus 10 taken along the line X-X in Figure 1B), by offsetting the optical sensor 25 from the locations L1 and L2 at which the support structure contacts the patient's tissue, the optical sensor 25 may be prevented from pressing against the vein 50.

In particular, as depicted in Figure 1C, when the support unit 23 is positioned against the tissue of the patient, the optical sensor 25 is offset from a plane P'-P' passing through the first L1 and second L2 points at which the tissue 55 of the patient contacts the support unit 23 (e.g. the first L2 and second L2 contact regions). Here, the optical sensor 25 is offset perpendicularly from the plane P'-P' by a distance D, wherein the distance D is equal to or great than the diameter of the vein 50.

In other words, the recess or channel 24 provided in the support unit 23 is adapted to be positioned at the target region of the patient's tissue. By providing the optical sensor 25 in the recess or channel 24, the optical sensor 25 is prevented from pressing against the target region of the tissue (when the recess/channel 24 is positioned so as to cover the target region of the tissue). The recess/channel 24, instead, provides a space or chamber 52 for the target region of the tissue to enter when the support unit 23 is placed against the patient's tissue, thereby preventing the target region of the tissue from being compressed (which may cause an underlying vein 50 to collapse for example). Thus, when the support unit 23 is positioned against the tissue 55 of the patient, the optical sensor 25, the support unit 23 and the tissue 55 of the patient combine to form a chamber 52, wherein the optical sensor 25 and the target region (e.g. the vein 50) of the tissue of the patient are positioned at opposite sides of the chamber 52 (e.g. facing each other across the chamber 52). Such a chamber 52 comprising the optical sensor 25 provides room for the vein 50 underlying the tissue, thereby preventing the vein 50 from being collapsed (which would otherwise block blood flow).

Also, referring to Figure 1D, there is depicted a modification to the embodiment illustrated in Figure 1C. More specifically, the Figure 1D illustrates an embodiment which further comprises a vacuum arrangement that is adapted to create a negative pressure in the chamber 52. Here, the support unit 23 is provided with tube or conduits 60 to enable air or liquid to be removed from the chamber 52, as illustrated by the arrows labelled "V". The creation of a negative pressure or vacuum in the chamber 52 may then cause the surface of the tissue 55 to be brought into contact with (or towards) the optical sensor 25, thereby minimizing or removing any air gap between the tissue 55 and the optical sensor 25 unit for improved sensing accuracy.

It is also noted that, in other embodiments (such as those employing an ultrasound sensor for example), the chamber may be filled with a fluid to improve coupling between the sensor unit and the tissue 55. In such an embodiment, air may be sucked out of the chamber 52 via a first channel or conduit and fluid may enter the chamber 52 via a second channel or conduit. Preferably, all the air should be sucked out, and so it may be preferable to arrange the first channel or conduit close to the sensor unit for example.

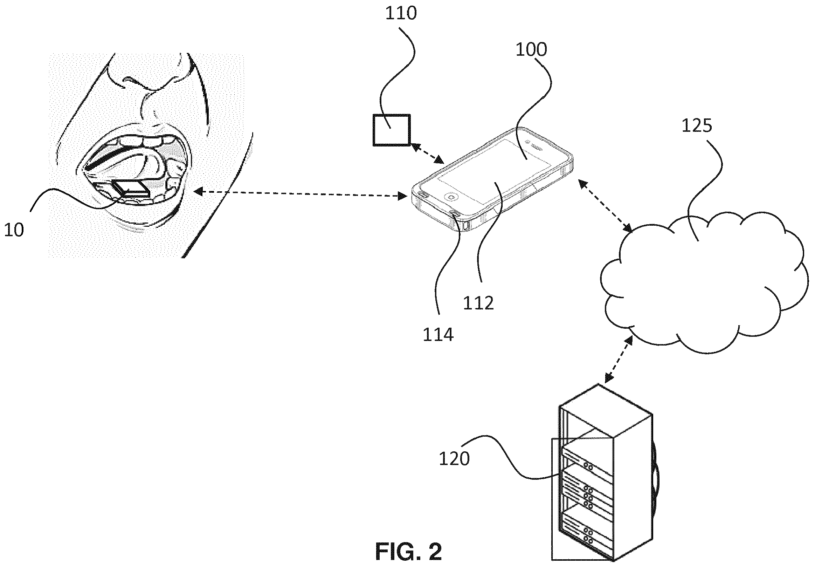

Turning now to Figure 2, there is illustrated an apparatus for monitoring hemodynamics (e.g. cardio-respiratory function) of a patient according to an embodiment. In such an embodiment, the apparatus comprises a sublingual apparatus 10 similar to that depicted in Figure 1 and a processing unit which is integrated in a portable computing device (e.g. a smartphone) 100. Using a built-in communication interface, the portable computing device 100 can receive signals from the apparatus 10 and other, supplementary sensors 110, 130 and the process the received signals in accordance with one or more data processing algorithms to determine a cardio-respiratory value of the patient. Further, using the conventional communication abilities of the portable computing device, the device can communicate with one or more databases so as to obtain information that may be used in determining or monitoring a cardio-respiratory value. Such user-specified information may enable unique traits, circumstances and/or conditions specific to the user or the environment to be accounted for when determining or monitoring a cardio-respiratory value.

Also, the display of the portable computing device 100 may be used to display a graphical user interface which communicates information about calculated cardio-respiratory functions to a user of the device.

In more detail, the embodiment of Figure 2 comprises comprise a client device 100, namely a smartphone 100, comprising data acquisition and processing components. The smartphone 100 is adapted to receive information from sublingual apparatus 10 positioned under a patient's tongue at the sublingual vein via a wireless communication link. Any suitable short-range or long-range communication links and/protocols may be employed.

The received sensor output signals from the sublingual apparatus 10 thus comprises data that may be used (e.g. processed in accordance with an algorithm so as to determine a value of a cardio-respiratory parameter. For example, the smartphone 100 of this example is adapted to implement a signal processing algorithm which identifies low-frequency optical sensor 25 output signal. Here, a RR is observed as the dominant frequency in the range between 0.08 Hz and 0.5 Hz. The smartphone thus implements a software application which monitors modulation in a low frequency optical sensor 25 output signal component which is indicative of the signal response to the deviations in respiration (caused by additional stress in the heart for example). Also, higher frequency variations in the optical sensor 25 output signal (for example in the range between 0.6Hz and 4Hz) are also used to provide information on average heart rate, heart rate variability, arrhythmias and SpO2, for example.

Furthermore, the smartphone 100 is also adapted to receive information from a supplementary sensor unit 110 which is adapted to sense a value of at least one of: movement; pressure; temperature; and sound. The smartphone 100 is adapted to analyze the determined cardio-respiratory value(s) in combination with the received supplementary sensor 110 output signal(s) to determine at least one of: a refined cardio-respiratory value; an indication of accuracy or reliability; a sleep state of the patient; an activity of the patient; and an indication of event occurrence. In this way, a context of the patient (such as their current activity or physical properties for example) can be taken into account in the process of determining and/or monitoring the cardio-respiratory function of the patient.

The smartphone 100 is also adapted to send and/or receive information to/from a remotely located server 120 via the Internet 125.

The information obtained by the smartphone 100 is processed to assess and identify factors which may influence the determined cardio-respiratory functions/parameters of the patient. By way of example, environmental information; patient information; and a limit value representative of an acceptable upper limit of a cardio-respiratory value may be used in the determination or monitoring of a cardio-respiratory value.

The information/data processing may be done by the smartphone 100, by the 'Cloud', or by any combination thereof. The embodiment of Figure 2 is therefore implemented as a distributed processing environment in which various types of information/data are processed so as to determine or monitor a cardio-respiratory function of the patient.

The smartphone 100 also comprises an output interface, namely a display 112 and speaker 114 arrangement, adapted to generate an output signal representative of the determined cardio-respiratory value/function. For example, if a dangerous cardio-respiratory value is determined or inferred, the user may be advised of the potential threat or danger and guided via voice or visual prompts to mitigate the threat/danger. The smartphone 100 is also adapted to receive (e.g. via its touch sensitive screen 112) a user input signal representative of at least one of: environmental information; patient information; and a limit value representative of an acceptable upper limit of a cardio-respiratory value.

The smartphone 100 therefore provides an interface which enables a user to further specify information or data that may be relevant for the purpose of determining or monitoring a cardio-respiratory value. Such user-specified information enables unique traits, circumstances and/or conditions specific to the user or the environment to be accounted for when determining or monitoring a cardio-respiratory value. Put another way, the smartphone 100 enables a user to further specify factors to be included in the determination of a cardio-respiratory value, e.g. by specifying a value or value range for a user attribute or activity. This provides many input options, increasing the flexibility and power of risk of cardio-respiratory monitoring.

Additionally, or alternatively, further environmental information and/or patient information may be provided by other sources or services. For example, local weather conditions and/or medical history data from a database of the server 120 can be used.

For example, in an exemplary implementation of the system of Figure 2, the server 120 comprises a data processor unit and is configured to transmit generated instructions for determining and/or displaying a determined cardio-respiratory value to a client device or communication network. In such a configuration, display instructions are made available by the server 120. A user of the smartphone 100 can therefore link with the server 120 to work with the system. In this way, data processing means are remotely located from the portable computing device 100, and a control signal can thus be communicated to the portable computing device 100 via a communication link (e.g. the Internet 125).

Accordingly, a user is provided with an appropriately arranged device that can receive and process information relating to a cardio-respiratory function of a patient. Embodiments may therefore enable a user to monitor a cardio-respiratory parameter over time using a portable computing device, such as a laptop, tablet computer, mobile phone, PDA, etc. The portable computing device 100 therefore provides a tool that enables the user, for instance, to monitor their cardio-respiratory function as they go about their normal activities. The user can obtain an understanding of their cardio-respiratory function, which then enables the user to continue or adapt their planned activities (depending on their tolerance to cardio-respiratory issues for example). Also, a medical professional, technician, researcher, etc. may have an appropriately arranged client device (such as a laptop, tablet computer, mobile phone, PDA, etc.) which is adapted to receive information relating to cardio-respiratory function of a monitored user (e.g. patient). In this way, a user can be provided with guidance at a personal level which takes account of the unique attributes and/or activities of the user, and/or the surrounding environment. This alleviates a need for close monitoring by medical professionals or caregivers, for example. It may also reduce a need for medical intervention or treatment (required as a result of repeated infection for example).

Dedicated data processing means can therefore be implemented at the server 120 for the purpose of determining cardio-respiratory value of a patient, thus relieving or reducing processing requirements at the portable computing device 100.

Thus, it will be understood that processing capabilities may therefore be distributed throughout the system in different ways according to predetermined constraints and/or availability of processing resources of specific embodiments.

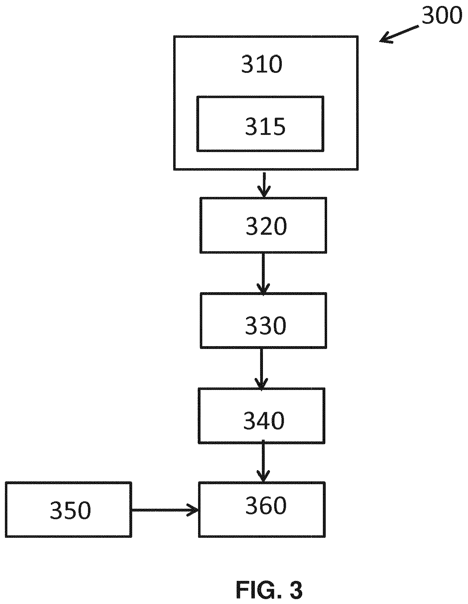

Turning now to Figure 3, there is depicted a flow diagram of a method 300 for monitoring hemodynamics of a patient according to an embodiment.

The method begins with step 310 of appropriately positioning equipment with respect to the patient. More specifically, the step 310 comprises positioning a support unit against the tissue of the patient so as to contact the tissue at first and second contact regions situated either side of a target region of the tissue of the patient. Here, the support unit supports a sensor unit adapted to detect light or sound from tissue of the patient and to generate sensor output signals based on the detected light or sound, and when the support unit is positioned against the tissue of the patient, the sensor unit is offset from a plane passing through the first and second contact regions and arranged to detect light or sound from the target region of the tissue of the patient.

Also, in this example, step 310 may comprises positioning the support unit such that when it is positioned against the tissue of the patient, the sensor unit, the support unit and the tissue of the patient combine to form a chamber with the sensor unit and the target region of the tissue of the patient being positioned at opposite sides of the chamber. Step 310 may then further comprise the step 315 of creating a negative pressure in the chamber (so as to bring the sensor unit into contact with the target region of the tissue for example).

Next, in step 320, the target region of the patient's tissue is illuminated with light from one or more light sources. Here, the step of illuminating the patient's tissue with light from a light source comprises controlling the one or more light sources to emit light having a wavelength within a first range of wavelengths and/or to emit light having a wavelength within a second, different range of wavelengths. More specifically, in this example, the first range of wavelengths comprises visible light and the second range of wavelengths comprises infra-red light. This may of course be different for other embodiments.

In step 330, light from the target region of the tissue is detected by the optical sensor, and the optical sensor then generates a sensor output signal based on the detected light and communicates the sensor output signal to a processing unit in step 340.

Also, in step 350, a supplementary sensor output signal is generated by a supplementary sensor and communicated to the processing unit. More specifically, the supplementary sensor of this example comprises an accelerometer such that the supplementary sensor output signal comprises information relating to the patient's body posture and the sensed movement of the patient.

Having received the sensor output signal and the supplementary sensor output signal, the processing unit then processes the received signals in step 360. Here, the processing unit processes the received signals in accordance with a data processing algorithm to determine both a cardio-respiratory value of the patient and at least one of: an indication of accuracy or reliability of the cardio-respiratory value of the patient; an activity and posture of the patient; and event detection and recognition.

More specifically, the sensor output signal is used to determine the cardio-respiratory value of the patient by identifying low-frequency variations in the sensor output signal. Also, the supplementary sensor output signal is used in combination with the determined cardio-respiratory value of the patient to determine at least one of an indication of accuracy or reliability of the cardio-respiratory value of the patient; an activity and posture of the patient; and event detection and recognition.

From the above description of the flow diagram in Figure 3, it will be understood that embodiments may provide a concept for monitoring hemodynamics (e.g. one or more cardio-respiratory functions) of a patient. The proposed concept may comprise positioning an optical sensor at (e.g. adjacent, next to, neighboring) a sublingual vein of the patient's tongue for example. Light transmitted through the sublingual vein may then be detected by the optical sensor and the detected light may be used (e.g. processed according to one or more algorithms) to determine a value of a cardio-respiratory functions of the patient. Determining the value of a cardio-respiratory function may also comprise taking account of historical information relating to previously determined values of the cardio-respiratory function of the patient. Data can be stored (e.g. in an on-board memory storage unit or in a remotely provision database) for logging of values and events, or can be streamed (e.g. using an on-board antenna/transmitter), which would also enable alarm generation. Also a hybrid solution is possible, in which data are, by default, stored locally but, in the case of a particular event the antenna may be used to send an alarm.

For such purposes, proposed concepts may employ (or be employed on) at least one processor.

Thus, there may be provided a computer program product downloadable from a communications network and/or stored on a computer readable medium and/or microprocessor-executable medium wherein the computer program product comprises computer program code instructions, which when executed by at least one processor, implement a method according to a proposed embodiment.

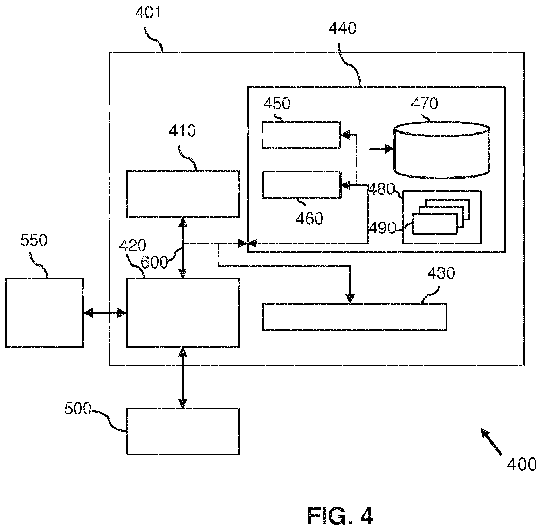

By way of example, as illustrated in Figure 4, embodiments may comprise a computer system 401, which may form part of a networked system 400. The components of computer system/server 401 may include, but are not limited to, one or more processing arrangements, for example comprising processors or processing units 410, a system memory 440, and a bus 600 that couples various system components including system memory 440 to processing unit 410.

Bus 600 represents one or more of any of several types of bus structures, including a memory bus or memory controller, a peripheral bus, an accelerated graphics port, and a processor or local bus using any of a variety of bus architectures. By way of example, and not limitation, such architectures include Industry Standard Architecture (ISA) bus, Micro Channel Architecture (MCA) bus, Enhanced ISA (EISA) bus, Video Electronics Standards Association (VESA) local bus, and Peripheral Component Interconnect (PCI) bus.

Computer system/server 401 typically includes a variety of computer system readable media. Such media may be any available media that is accessible by computer system/server 401, and it includes both volatile and non-volatile media, removable and non-removable media.

System memory 440 can include computer system readable media in the form of volatile memory, such as random access memory (RAM) 450 and/or cache memory 460. Computer system/server 401 may further include other removable/non-removable, volatile/non-volatile computer system storage media. By way of example only, storage system 440 can be provided for reading from and writing to a non-removable, non-volatile magnetic media (not shown and typically called a "hard drive"). Although not shown, a magnetic disk drive for reading from and writing to a removable, non-volatile magnetic disk (e.g., a "floppy disk"), and an optical disk drive for reading from or writing to a removable, non-volatile optical disk such as a CD-ROM, DVD-ROM or other optical media can be provided. In such instances, each can be connected to bus 600 by one or more data media interfaces. As will be further depicted and described below, memory 440 may include at least one program product having a set (e.g., at least one) of program modules that are configured to carry out the functions of embodiments of the invention.

Program/utility 480, having a set (at least one) of program modules 490, may be stored in memory 440 by way of example, and not limitation, as well as an operating system, one or more application programs, other program modules, and program data. Each of the operating system, one or more application programs, other program modules, and program data or some combination thereof, may include an implementation of a networking environment. Program modules 490 generally carry out the functions and/or methodologies of embodiments of the invention as described herein.

Computer system/server 401 may also communicate with one or more external devices 500 such as a keyboard, a pointing device, a display 550, etc.; one or more devices that enable a user to interact with computer system/server 401; and/or any devices (e.g., network card, modem, etc.) that enable computer system/server 401 to communicate with one or more other computing devices. Such communication can occur via Input/Output (I/O) interfaces 420. Still yet, computer system/server 401 can communicate with one or more networks such as a local area network (LAN), a general wide area network (WAN), and/or a public network (e.g., the Internet) via network adapter 430. As depicted, network adapter 430 communicates with the other components of computer system/server 401 via bus 600. It should be understood that although not shown, other hardware and/or software components could be used in conjunction with computer system/server 401. Examples, include, but are not limited to: microcode, device drivers, redundant processing units, external disk drive arrays, RAID systems, tape drives, and data archival storage systems, etc.

Thus, there is proposed a concept for providing guidance regarding cardio-respiratory function at a personal level which employs optical sensing of a central-site vein in response to temporary airway pressure changes. By sensing variations in blood volume in the central site vein in response to temporary airway pressure changes, venous information of the person may be obtained.

At this point, it is noted that the above described embodiments are merely example embodiments and that several extensions thereto and/or variations may be made.

For example, several types of supplementary monitoring/sensing devices can be envisaged, including clip-on devices, smart textiles, mouth inserts, etc.

Data from the system may be combined with personal data on health and well-being to generate a personal profile of "safe" and "risky" activities, locations and interactions. Data may also be transmitted for the benefit of other peer users or patients interested in cardio-respiratory functions, and such data could serve as input to complication avoidance software.

Other suitable extensions and variations to the above disclosed embodiments will be apparent to the skilled person.

For instance, although embodiments have been described in relation to sublingual vasculature, proposed concepts may be extended to other veins (e.g. veins that may provide right-heart side and respiratory information, which are in particular veins that drain into the internal jugular vein, such as particular veins in the nose and ear). Embodiments may also apply to venous information in general, which for example includes the external jugular vein and veins that drain into the external jugular vein. In a broad sense, embodiments may be applied to any veins or vasculature, including those in the lower arm, and may be particular useful for veins lying close to the surface of patient tissue (which may otherwise be squeezed or compressed by pressing conventional apparatus of the tissue).

Further, embodiments may be adapted to implement flexible thresholds that can be adapted according to user and/or with respect to time. In this way, it may be possible to have more or less strict versions of algorithms used to create alerts or notifications.

Also, also mentioned above, the optical sensor may comprise several light sources (e.g. LEDs) and/or several photo detectors. They may be arranged in an array-like structure. The optical sensor could also comprise a CCD chip or use laser-speckle technique.

In another embodiment, the optical sensor may be a (remote) camera, for example a camera that a GP has in his hand. The patient may be asked to lift his/her tongue up (or have his/her tongue lifted up by an additional device) in order to make the area around the sublingual vein visible to the camera.

A preferred implementation may be to only inform a user when a cardio-respiratory issue or anomaly is detected. This may help to ensure a seamless solution without inhibiting social interaction.

The proposed concept has the advantage that a network of portable computing device with monitoring and/or communication functions can be easily transformed into a cardio-respiratory function monitoring system.

Aspects of the present invention may be embodied as a cardio-respiratory function monitoring method or system at least partially embodied by a portable computing device or distributed over separate entities including a portable computing device. Aspects of the present invention may take the form of a computer program product embodied in one or more computer-readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. Such a system, apparatus or device may be accessible over any suitable network connection; for instance, the system, apparatus or device may be accessible over a network for retrieval of the computer readable program code over the network. Such a network may for instance be the Internet, a mobile communications network or the like. More specific examples (a non-exhaustive list) of the computer readable storage medium may include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of the present application, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, etc., or any suitable combination of the foregoing.

Computer program code for carrying out the methods of the present invention by execution on the processor 110 may be written in any combination of one or more programming languages, including an object oriented programming language such as Java, Smalltalk, C++ or the like and conventional procedural programming languages, such as the "C" programming language or similar programming languages. The program code may execute entirely on the processor 110 as a stand-alone software package, e.g. an app, or may be executed partly on the processor 110 and partly on a remote server. In the latter scenario, the remote server may be connected to the head-mountable computing device 100 through any type of network, including a local area network (LAN) or a wide area network (WAN), or the connection may be made to an external computer, e.g. through the Internet using an Internet Service Provider.

Aspects of the present invention are described above with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions to be executed in whole or in part on the data processor 110 of the cardiopulmonary resuscitation coordination system including portable computing device, such that the instructions create means for implementing the functions/acts specified in the flowchart and/or block diagram block or blocks. These computer program instructions may also be stored in a computer-readable medium that can direct the cardiopulmonary resuscitation guidance system including the portable computing device to function in a particular manner.

The computer program instructions may, for example, be loaded onto the portable computing device 100 to cause a series of operational steps to be performed on the portable computing device 100 and/or the server 120, to produce a computer-implemented process such that the instructions which execute on the portable computing device 100 and/or the server 120 provide processes for implementing the functions/acts specified in the flowchart and/or block diagram block or blocks. The computer program product may form part of a patient monitoring system including a portable computing device.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. Apparatus (10) for monitoring hemodynamics of a patient, the apparatus comprising:
a sensor unit (22) adapted to detect light or sound from tissue of the patient and to generate sensor output signals based on the detected light or sound; and
a support unit (23) adapted to support the sensor unit and comprising a recess (24),
wherein the support unit is adapted to be positioned against the tissue of the patient,
and wherein, when the support unit is positioned against the tissue of the patient, the recess is adapted to be positioned to cover a target region of the tissue of the patient and the sensor unit is adapted to detect light or sound from the target region of the tissue of the patient.

2. The apparatus of claim 1, wherein, when the support unit (23) is positioned against the tissue of the patient, the recess (24) and the target region tissue (55) of the patient combine to form a chamber (52) and the sensor unit (22) is positioned to face the target region of the tissue of the patient.

3. The apparatus of claim 2, further comprises a vacuum arrangement (60) adapted, when the support unit is positioned against the tissue of the patient, to create a negative pressure in the chamber.

4. The apparatus of any preceding claim, wherein, when the support unit (23) is positioned against the tissue of the patient, the support unit contacts the tissue at first (W1) and second (W2) contact regions situated either side of the target region of the tissue of the patient and the sensor unit is offset from a plane (P'-P') passing through the first and second contact regions.

5. The apparatus of claim 4, wherein the target region of the tissue comprises a target vein (50) and wherein the recess has a depth substantially equal to the diameter of the target vein.

6. The apparatus of any preceding claim, wherein the support unit (23) is adapted to be positioned at vasculature draining into the internal jugular vein of the patient.

7. The apparatus of claim 6, wherein the support unit (23) is adapted to be positioned at sublingual vasculature of the patient's tongue, and wherein the target region of the tissue of the patient comprises sublingual vasculature, and preferably wherein the recess has a depth in the rage of 1 mm to 4 mm.

8. The apparatus of any preceding claim, further comprising a supplementary sensor module (110) and comprising a sensor arrangement adapted to sense a value of at least one of: movement; pressure; temperature; and sound and to generate a supplementary sensor output signal based on the sensed value(s).

9. The apparatus of any preceding claim, further comprising a processing unit (100) adapted to receive at least one of the sensor output signal and the supplementary sensor output signal and to process at least one of the received signals in accordance with one or more data processing algorithms to determine a cardio-respiratory value of the patient,
and wherein the cardio-respiratory value comprises a value of at least one of:
respiration rate; respiration rate variability; onset of inspiration; onset of expiration; duty cycle of respiration; Cheyne-Stokes respiration; obstructive sleep apnea; central sleep apnea; obstructive flow limitation; transmural pressure; blood accumulation; coughing; paced breathing; and pursed breathing.

10. The apparatus of claim 9, wherein the data-processing algorithm is adapted to identify low-frequency variations in the sensor output signal.

11. The apparatus of any preceding claim, wherein the sensor unit (22) comprises at least one of: a photoplethysmography sensor; a laser speckle sensor; a laser Doppler sensor; an ultrasound sensor; and a camera.

12. A mouthpiece comprising:
a mouthpiece unit adapted to be positioned, in use, in the mouth of a patient; and
apparatus for monitoring cardio-respiratory function of a patient according to any preceding claim,
and preferably wherein the sensor unit is adapted to be movable with respect to the mouthpiece unit.

13. A method for monitoring hemodynamics of a patient, the method comprising:
positioning (310) a support unit against the tissue of the patient, wherein the support unit comprises a recess and supports a sensor unit, the sensor unit being adapted to detect light or sound from tissue of the patient and to generate sensor output signals based on the detected light or sound, and wherein when the support unit is positioned against the tissue of the patient, the recess is adapted to be positioned to cover a target region of the tissue of the patient;
detecting (330) light or sound from the target region of the tissue of the patient; and
generating (340) a sensor output signal based on the detected light or sound.

14. The method of claim 13, wherein, when the support unit is positioned against the tissue of the patient, the recess and the target region of the tissue of the patient combine to form a chamber with the sensor unit being positioned to face the target region of the tissue of the patient, and optionally further comprising:
creating (315) a negative pressure in the chamber.

15. A computer program product downloadable from a communications network and/or stored on a computer readable medium and/or microprocessor-executable medium wherein the computer program product comprises computer program code instructions, which when executed by at least one processor, implement a method as claimed in claim 13 or 14.
